# EUROPEAN PATENT APPLICATION

(11) **EP 4 306 953 A1**
(43) Date of publication of application: **17.01.2024**
(21) Application number: 22382660.3
(22) Date of filing: 12.07.2022
(51) Int. Cl.: G01N 33/50, A61K 31/325, A61K 31/427, A61K 31/635, A61K 45/06, A61P 35/00, G01N 33/574

(54) **REAL-TIME TRACKING OF APOPTOSIS**

(71) Applicant: Fundació Institut de Bioenginyeria de Catalunya (IBEC), 08028 Barcelona (ES); Universitat de Barcelona, 08028 Barcelona (ES); Institució Catalana De Recerca I Estudis Avançats (ICREA), 08010 Barcelona (ES)
(72) Inventor: MONTERO, Juan, J., 08028 Barcelona (ES); MANZANO, Albert, 08028 Barcelona (ES); SAMITIER, Josep, 08028 Barcelona (ES); RAMON, Javier, 08028 Barcelona (ES); ORTEGA, María, Alejandra, 08028 Barcelona (ES)
(74) Representative: Hoffmann Eitle

(57) **Abstract**

The present invention provides compositions, kits and methods allowing the prediction of the cytotoxicity or cell protection of a therapeutic agent without altering the permeability of the tested cells.

## Description

### Field of the invention

The present invention refers to anti-cancer therapy effectiveness prediction. More particularly, the present invention provides a combination of anti-apoptotic inhibitors useful in predicting the sensitivity of a subject to an anti-cancer therapy as well as kits and devices containing thereof.

### Background of the invention

As more targeted therapies are approved for different types of cancer, there is a growing need for predictive biomarkers so that these therapies can be directed to patients who will most benefit from them. Unfortunately, the current biomarkers available for cancer therapy are not sufficient.

Recently, the development of tyrosine kinase inhibitors (TKI) has improved treatment in patients with advanced disease. For example, detection of mutations in EGFR has been successfully used as a biomarker for initial therapy with EGFR inhibitors. However, many targeted agents lack genetic predictive markers. Furthermore, resistance to these drugs frequently emerges, and it is often not clear what treatment is best given following this emergence of resistance, given the variety of mechanisms for resistance.

As precision medicine for cancer emerges, new predictive biomarkers to assess therapies' effectiveness in patients are desperately needed. Beyond standard molecular analyses, functional assays are currently developed to guide clinical decisions.

In this regard, it has been reported the use of the so-called dynamic BH3 profiling or DBP to directly evaluate therapies.

This method is based in catalyzing the process of apoptotic cell death by using synthetic BH3 peptides. However, the actual methods reported in the method require to permeabilize cells with digitonin to allow the BH3 peptides internalization and perform their pro-apoptotic activity. This represents a clear limitation of the assay, since it forces to fix the cells (thus killing them) after the incubation with the peptides allowing to visualize only one timepoint. See, for instance US20220163510.

Therefore, despite the efforts made, there is still the need for further tools to appropriately predict response to anti-cancer agents.

### Summary of the Invention

The present inventors have identified a combination of anti-apoptotic inhibitors which can provide efficient and sensitive predictive information about the response of a subject to a particular therapy or therapies.

In particular, the present inventors have designed a combination of molecules providing an inhibitory effect towards BCL-2, BCL-xL, and MCL-1. As it is shown below, the inhibitors forming the combination of the invention can efficiently penetrate the cancer cell without damaging it. And, therefore, no permeabilization agent is required.

Furthermore, the inventors have found that the apoptotic state induced by the combination of inhibitors of the invention is at least equal or even higher than the apoptotic effect provided by the DBP protocol provided in the prior art.

Therefore, the present invention provides a combination which is at least as efficient as the BH3 peptides/digitonin already reported in the prior art DBP protocols in determining apoptotic priming, with the remarkable advantage that the cell viability of the test cell population samples is not negatively affected because no cell permeabilization is required in the present case.

The above means a great advance with respect to the up to now DBP methodology disclosed in the prior art.

On one hand because in the prior art two steps were required to obtain the priming effect of the apoptosis: (1) increase the permeabilization of the cell membrane by administering digitonin, and (2) contact the cell with synthetic BH3 peptides to catalyze apoptosis.

Advantageously, with the combination of the invention the permeabilization step would be avoided allowing the continuous monitoring of living cells.

On the other hand, one of the main concerns in the management of cancer is not only the prediction of the response when the subject is intended to initiate an anticancer therapy, but how that subject could react with a prolonged anticancer therapy (e.g., development of resistance to the therapy).

An important advantage of using the combination of inhibitors of the present invention is that, when the combination is administered, it can prime a high-degree apoptotic state without negatively affecting to the cancer cell integrity. This allows, with a single test sample, a real-time monitoring of the evolution of the cancer cells with prolonged therapies. This information being of great usability in the appropriate management of the cancer disease at the different stages, in the prediction of a potential onset of resistance towards the treatment and testing several anti-cancer therapies over time.

Altogether, the combination provided by the present invention means a great advance in the efficient management of anti-cancer therapy.

Thus, in a first aspect the present invention provides a combination comprising a BCL-2 inhibitor, a BCL-xL inhibitor and a MCL-1 inhibitor.

In a second aspect the present invention provides a kit comprising the combination of inhibitors as defined in the first aspect of the invention.

The present inventors have developed methods wherein it is possible to predict how sensitive a subject is towards a particular candidate drug(s), which is based on one or more antiapoptotic BCL-2 family inhibitors having a molecular lower than 2.0 KDa.

Remarkably, in the context of the present invention the inhibitors are used as reagents and not as therapeutic drugs.

The methods of the present invention are based on measuring how close a cell is to the threshold of programmed cell death (i.e. apoptosis), also known as measuring how "primed" the cancer cell is for death. The methods of the invention allows the identification of drugs that move cells closer to the threshold of programmed cell death (increase apoptotic priming the most). These methods of the invention can be applied to individual clinical cancer samples, so that those drugs that move the cells in that sample closest to the threshold of programmed cell death for that individual sample can be readily identified. The drugs so identified are those most likely to provide clinical benefit to the subject from which the sample was derived. Therefore, the invention provides a methods of personalizing therapy for individual cancer patients based on using one or more small molecules inhibitors of BCL-2 anti-apoptotic family.

Thus, in a third aspect the present invention provides a method for predicting the cytotoxicity of a therapeutic agent towards a cell, the method comprising: (a) contacting an isolated test cell population to the test therapeutic agent; (b) contacting the cell population resulting from step (a) to one or more anti-apoptotic BCL-2 family inhibitors at a particular concentration, the one or more anti-apoptotic BCL-2 family inhibitors having a molecular weight below 2 KDa, particularly below 1.5 KDa, particularly below 1 KDa ; (c) determining an apoptotic priming value; (d) obtaining a reference apoptotic priming value by contacting another isolated cell population sample from the subject with the one or more anti-apoptotic BCL-2 family inhibitors used in previous step (b); and (e) comparing both priming values; wherein: step (d) is performed in the absence of the therapeutic agent; and the process is performed in the absence of a cell permeabilizing agent; and wherein a reduction in the priming value from the test cell portion compared to that in the reference cell portion indicates that the therapeutic agent is cytotoxic for the cell. The resulting change in apoptotic priming between treated versus untreated after exposure to the inhibitor(s) of the BCL-2 family, results in a change in apoptotic priming upon treatment (normally defined as Δ% priming) that is indicative of the treatment's capacity to engage apoptosis and eliminate target cells. Since this invention permits to perform this determination on living cancer cells, several treatments can be administered and monitored over time as a real-time tracking of apoptotic engagement.

In a fourth aspect the present invention provides a method for predicting cell protection of a therapeutic agent, the method comprising: (a) contacting an isolated test cell population to the test therapeutic agent; (b) contacting the cell population resulting from step (a) to one or more anti-apoptotic BCL-2 family inhibitors at a particular concentration, the one or more antiapoptotic BCL-2 family inhibitors having a molecular weight below 2 KDa, particularly below 1.5 KDa, particularly below 1 KDa; (c) determining an apoptotic priming value; (d) obtaining a reference apoptotic priming value by contacting another isolated cell population sample from the subject with the one or more anti-apoptotic BCL-2 family inhibitors used in previous step (b); and (e) comparing both priming values; wherein: step (d) is performed in the absence of the therapeutic agent; and the process is performed in the absence of a cell permeabilizing agent; and wherein an increase in the priming value from the test cell portion compared to that in the reference cell portion indicates that the therapeutic agent protects the cell.

### Description of Drawings

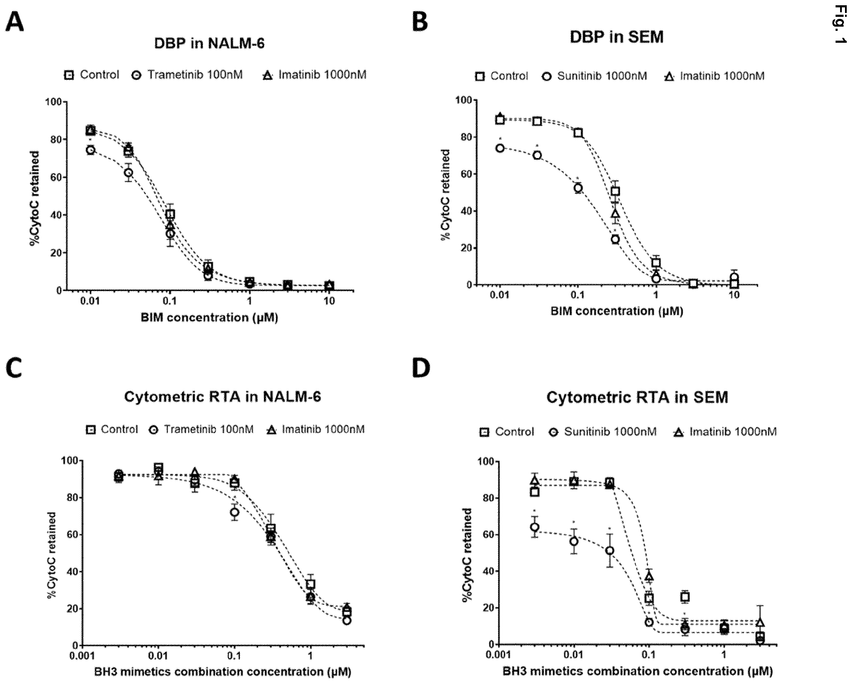

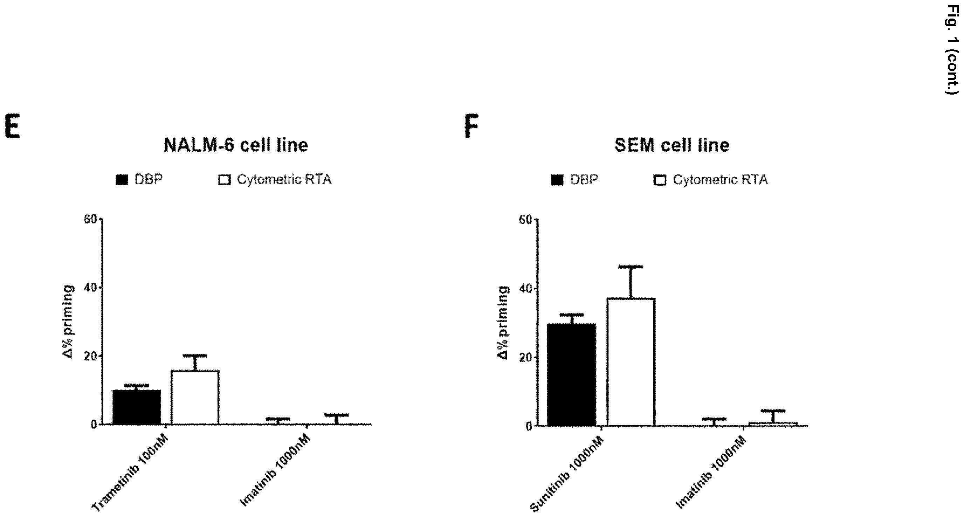

**Fig. 1****.** Represents the % of cytochrome c release (Y-axis) after treatment with anti-cancer treatments for 16 hours with BIM peptide (comparative purpose, standard DBP) in A) NALM-6 and B) SEM cell lines; after treatment for 16 hours with anti-cancer drugs using the combination of the invention as apoptotic inducer and anti-cytochrome C antibody to identify apoptotic from non-apoptotic cells using flow cytometry in C) NALM-6 and D) SEM cell lines. Figures 1E and F represents Δ%Priming between the standard DBP and the method of the invention in E) NALM-6 and F) SEM cell lines.

Squares= reference; cercles= Trametinib 100nM in (A), and (C); or Sunitinib 1000 nM in (B), and (D); Triangles= imatinib 1000 nM

Black bar represents Δ%priming with DBP, and white bar represents Δ%priming with cytometric RTA in (E) and (F).

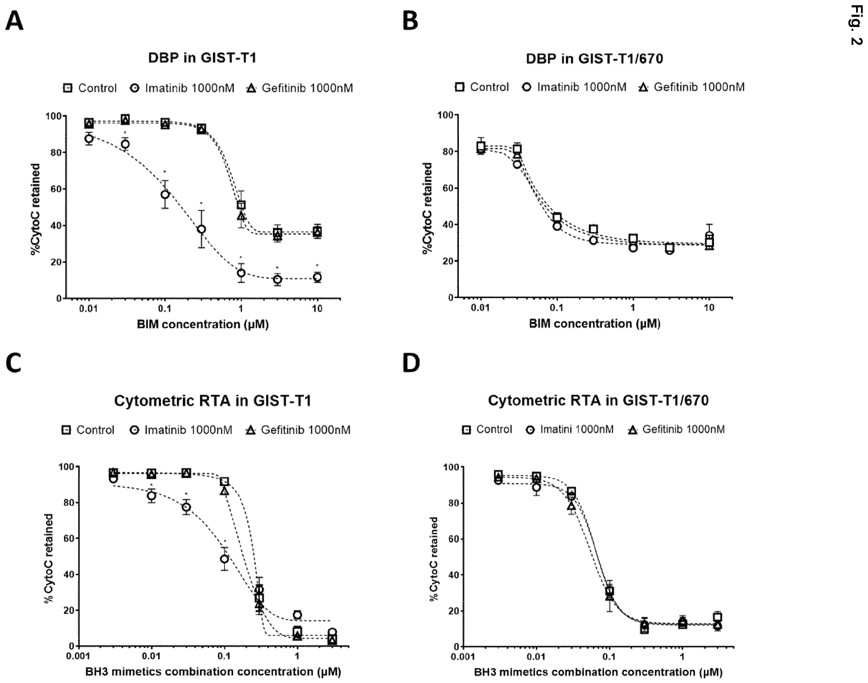

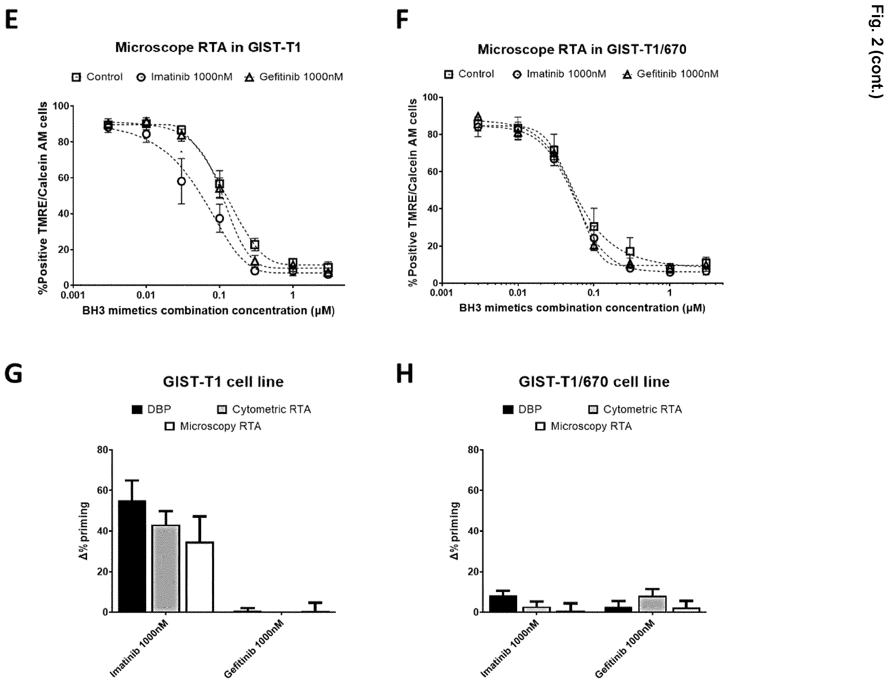

**Fig. 2****.** Represents the % of cytochrome c release (Y-axis) after treatment with anti-cancer treatments for 16 hours with BIM peptide (comparative purpose, standard DBP) in A) GIST-T1 and B) GIST-T1/670 cell lines. RTA after treatment for 16 hours with anti-cancer drugs using the combination of BH3 mimetics of the invention as apoptotic inducer and anti-cytochrome C antibody to identify apoptotic from non-apoptotic cells using flow cytometry in C) GIST-T1 and D) GIST-T1/670 cell lines. RTA uses the combination of BH3 mimetics as an inductor of apoptosis and TMRE to identify apoptotic and non-apoptotic cells using a fluorescence microscope in E) NALM-6 and F) SEM cell lines. Comparison of Δ%Priming between the normal DBP and the cytometric RTA using the combination of BH3 mimetics in G) GIST-T1 and H) GIST-T1/670 cell lines.

Squares= reference; cercles= imatinib 1000 nM; Triangles= gefitinib 1000 nM

Black bar represents Δ%priming with DBP, grey bar represents Δ%priming with cytometric RTA and white bars represent Δ%priming with microscope RTA in (E) and (F).

### Detailed Description

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

For the purposes of the present invention, any ranges given include both the lower and the upper end-points of the range.

As used herein, the meaning of the term "comprising" encompasses three alternatives, namely "comprising", "consisting of" and "consisting essentially of".

The present invention provides in a first aspect of the invention a combination comprising molecules inhibiting BCL-2, BCL-xL, and MCL-1.

In the context of the present invention, the combination can include one molecule specific for each one of the targets, that is, the combination will include 3 molecules. In other cases, the combination can include a molecule which exert a dual effect on two of the targets (for example BCL-2/BCLxL inhibitors). In this alternative case, the combination will include two molecules for inhibiting BCL-2, BCLxL and MCL-1: one with the dual effect and a second one specific for the other target.

The intrinsic pathway, also known as the mitochondrial pathway, is regulated by the BCL2 family of proteins. This family has different members that can be classified based on structure, function, and BCL2 homology (BH) domains (11, 12): activator members, antiapoptotic members, sensitizers, and effectors.

In the context of the present invention, the molecules to be incorporated in the combination are those which bind with high affinity and specificity to the hydrophobic groove of the antiapoptotic protein targets, inhibiting them.

There are already known inhibitors of each one of the three families (BCL-2, BCLXL and MCL-1). By the way of illustration, some of them are explained in detailed below.

### Molecules inhibiting BCL-2

The first molecule described to target BCL2 was HA14-1, which showed *in vitro* and *in vivo* activity alone or in combination with cytotoxic therapy.

Abbott Laboratories (now AbbVie) developed ABT-737, the first on-target specific BH3 mimetic inhibiting BCL2, BCLXL, and BCLW. This compound was further refined to improve its oral bioavailability, leading to the analogue ABT-263 (navitoclax).

Navitoclax showed promising results in multiple blood cancers, particularly chronic lymphocytic leukemia (CLL), which express high levels of BCL2.

To reduce the risk of thrombocytopenia, AbbVie developed a selective BCL2 inhibitor called ABT-199 (venetoclax). The clinical response of patients with CLL to venetoclax was evidence of impressive anticancer activity, leading to rapid debulking and even instances of tumor lysis syndrome.

Other illustrative non-limitative examples of BCL-2 inhibitors are APG-1252, BGB-11417, S55746 or SPC2996, among others.

The Examples provided below are based on a combination comprising ABT-199 as BCL-2 inhibitor. The same results as those provided by ABT-199 could also be obtained with any of the above-cited inhibitors of the same family because they have a so small size that they could also diffuse through cell membranes.

### Molecules inhibiting BCLxL

Selective BCLXL inhibitors have also been developed for their potential anticancer activity. One of the first compounds shown to be highly selective was WEHI-539, which was also effective against solid tumors. More recent derivatives of this first BCLXL inhibitor, such as A-1155463 or A-1331852 have also been reported.

More recent derivatives of this first BCLXL inhibitor, such as A-1155463 or A-1331852, have also been studied, with promising preclinical results.

In particular, the latter holds great promise due to its oral bioavailability. In contrast, a new strategy targeting BCLXL degradation using a proteolysis-targeting chimera (PROTAC; DT2216), which has unique selectivity to target this antiapoptotic protein in tumor cells but not in platelets, is now under evaluation, with clinical potential and would also form part of the scope of the invention.

The Examples provided below are based on a combination comprising A-1331852 as BCL-xL inhibitor. The same results as those provided by A-1331852 could also be obtained with any of the above-cited inhibitors of the same family because they have a so small size that they could also diffuse through cell membrane without any effort.

### Molecules inhibiting MCL1

The observation that the MCL1 antiapoptotic protein is commonly used by cancer cells to evade apoptosis has also stimulated the development of novel targeted therapies.

One of the first selective inhibitors was A-1210477, presenting excellent in vitro results in hematologic malignancies and solid tumors, such as breast and lung cancer cell lines, particularly in combination with navitoclax. Encouraged by the anticancer action of these molecules, a new generation of promising small-molecule MCL1 antagonists is now in clinical development. These include S64315/MIK665, AZD-5991, PRT1419, and AMG-176, among others, which are currently being explored in clinical trials (NCT02992483, NCT04629443, NCT03013998, NCT02675452, NCT04178902, NCT04543305, and others), mostly in hematologic malignancies. Other compounds that inhibit or degrade MCL1 have also demonstrated activity in preclinical models. Given that MCL1 is a short-lived protein, indirect targeting by CDK9 inhibitors, such as alvocidib, AZD4573 (NCT03263637), or voruciclib (NCT03547115), or protein selective degradation approaches have emerged as alternative strategies. BFL1/A1 can also be similarly targeted using CDK9 inhibitors or dual inhibitors of MCL1 and BFL1.

S63845 has also been reported as a very selective MCL1 inhibitor, with no cross-reactivity towards BCL2 or BCLxL.

The Examples provided below are based on a combination comprising S63845 as MCL1 inhibitor. The same results as those provided by S63845 could also be obtained with any of the above-cited inhibitors of the same family because they have a so small size that they could also diffuse through cell membrane without any effort.

The skilled person, just using their general knowledge can easily pick the inhibitor from each one the three groups, or use promiscuous anti-apoptotic inhibitors, that is small molecules that inhibit several anti-apoptotic proteins at once such as ABT-263, APG-1252, AZD0466, and others) and combine them to obtain similar results.

In one embodiment of the first aspect of the invention, optionally in combination with any of the embodiments provided above or below, the BCL2 inhibitor is ABT-199.

In another embodiment of the first aspect of the invention, optionally in combination with any of the embodiments provided above or below, the BCLXL inhibitor is A-1331852.

In another embodiment of the first aspect of the invention, optionally in combination with any of the embodiments provided above or below, the MCL1 inhibitor is one which binds to BH3-binding groove of MCL1, particularly S63845.

In one embodiment of the first aspect of the invention, optionally in combination with any of the embodiments provided above or below, the combination comprises ABT-199, A-1331852 and S63845. In one embodiment of the first aspect of the invention, optionally in combination with any of the embodiments provided above or below, the combination consists of ABT-199, A-1331852 and S63845.

In another embodiment of the first aspect of the invention, optionally in combination with any of the embodiments provided above or below, the combination comprises one or more other types of anti-apoptotic BCL-2 family inhibitors, such as activators of pro-apoptotic members of the BCL-2 family, particularly BAX, BAK1, BIM, BID and BBC3 (commonly referred to as PUMA), and inhibitors of anti-apoptotic members of the same family, such as BCL-2, BCL-XL, BCL-W, and MCL-1. These further inhibitors have a molecular weight below 2 KDa, more particularly below 1.9, below 1.8, below 1.7, below 1.6, below 1.5, below 1.4, below 1.3, below 1.2, below 1.1 or below 1.0 KDa.

Activator proteins (BIM, BID and PUMA) bind and directly activate BAX and/or BAK1, the central effectors of apoptosis, which subsequently undergo conformational changes and oligomerize, forming a pore in the mitochondrial membrane and causing MOMP.

The function of sensitizer proteins (BAD, PMA- induced protein 1 (commonly referred to as NOXA), BIK, BMF and HRK) is that of inhibiting anti- apoptotic proteins and/or displacing activators (such as BIM or monomers of BAX or BAK1) that could ultimately lead to apoptosis. Anti- apoptotic members of the BCL-2 family have four BCL-2 homology domains forming a binding groove that sequesters activators or sensitizers as well as BAX and BAK1. The BH3-only proteins PUMA, NOXA, BID and BIM have a strong affinity for and inhibit MCL1, whereas BAD has a stronger affinity for BCL-2, BCL-W and BCL-XL than for MCL1.

In the context of the present invention, a "anti-apoptotic BCL-2 family inhibitor" is understood as any well-known small molecule which either directly (binding to BCL-2) or indirectly negatively affects to BCL-2 cell activity, either because it BCL-2 is eliminated/degraded or because, even being produced, its action is binding-inhibited.In another embodiment of the first aspect of the invention, optionally in combination with any of the embodiments provided above or below, the combination comprises one or more other types of anti-apoptotic BCL-2 family inhibitors, other than the ones belonging to BCL-2 inhibitors, BCL-xL inhibitors and/or MCL-1 inhibitors referred in claim 1.The preparation of the combination is performed routinely, mixing the small molecules under agitation. These molecules have to be added at a concentration at which they provide the inhibitory effect on the target. This concentration will usually correspond to the one recommended by the manufacturing company. But, the skilled person can precisely adjust, without any effort, the concentration of the particular inhibitory molecule, in case it was necessary, just using routine tests. For example, based on the Ki values.

Examples of anti-apoptotic BCL-2 family inhibitors include, but are not restricted to, HA14-2, Obatoclax, Gossypol (AT-101), Oblimersen, ABT-737, ABT-263 (navitoclax), ABT-199 (venetoclax), APG-1252, APG-2575, AZD0466, BGB-11417, NU-0129, S55746/BCI-201, SPC2996, WEHI-539, A1155463, A1331852, DT2216, S-055746, A-1210477, AMG-176, AMG-397, AZD-5991, PRT1419, S64315/MIK665, S63845, MIM1, VU661013, GDC-0941, and others.

### Kits

All the embodiments provided above under the first aspect of the invention, related to the combination of inhibitors, are also embodiments of the kit of the second aspect of the invention.

In one embodiment of the second aspect of the invention, optionally in combination with any of the embodiments provided above or below, the kit comprises a vial containing mitochondrial buffer and instructions for use.

In another embodiment of the second aspect of the invention, optionally in combination with any of the embodiments provided above or below, the combination comprises one or more other types anti-apoptotic BCL-2 family inhibitors, as those referred above, such as activators of pro-apoptotic members of the BCL-2 family, such as BAX, BAK1, BIM, BID and BBC3 (commonly referred to as PUMA), and inhibitors of anti-apoptotic members of the same family, such as BCL-2, BCL-XL, BCL-W, and MCL-1. Illustrative examples have been provided above.

In another embodiment of the second aspect of the invention, optionally in combination with any of the embodiments provided above or below, the kit comprises each one of the inhibitors in a separate compartment with instructions to prepare the combination of the invention. Alternatively, in another embodiment of the second aspect of the invention, optionally in combination with any of the embodiments provided above or below, the kit already comprises the combination of the inhibitors of the first aspect of the invention.

In another embodiment of the third aspect of the invention, optionally in combination with any of the embodiments provided above or below, the kit is a microfluidic device.

Microfluidic devices are easily manufacture, are low-cost, robust and can be used in the long term to routinely perform predictive cancer therapy response assays in hospitals at a minimal cost. Moreover, since the cell requirement is lower than current technologies, these analyses can be done in non-surgically obtained biopsies such as fine-needle aspirates. From a clinical standpoint this approach may represent a breakthrough on how to treat cancer patients and can greatly improve clinical outcomes, even for the most complicated cases (relapsed/refractory/metastatic tumors).

Any of the microfluidic devices commercially available can be used in the context of the invention, just following the manufacturer's instructions.

Chemical gradients play a key role in many biological processes and regulate a number of cellular functions *in vivo.* Indeed, there are several examples of gradient-dependent phenomena in nature such as, for example, chemotaxis, i.e. the cell migration triggered by establishing a gradient. Moreover, chemical gradients have been shown to affect various cell behaviors, such as tumor genesis.

Therefore, it is evident that studying and possibly recreating these conditions *in vitro* is crucial in life science related studies.

In one embodiment of the second aspect of the invention, the kit is a microfluidic device including means to generate a gradient either logarithmic or lineal, particularly a logarithmic gradient, of the combination of inhibitors object of the present invention. This allows the replication of the dose-curve.

The two most common ways of creating chemical gradients in microfluidics are: (i) the use of *ad hoc* designed microchannels with at least two inlets" in which adjacent streams containing different concentrations of the chemical of interest (i.e., the combination of inhibitors as defined in the first aspect of the invention) are mixed in order to generate the gradient, and (ii) the use of the ability of cells to secrete and consume biochemicals in their vicinity combined with slow perfusion to create gradients in a direction parallel to the direction of fluid flow.

The skilled person in the art already can select, among the commercial ones with the gradient function, the most appropriate. If they would like to design their own device, with a gradient, then, making use of their general knowledge, they would be capable of selecting among the different means, the one more appropriate to generate such gradient. In one embodiment, optionally in combination with any of the embodiments provided above or below, the microfluidic device includes a gradient generator, particularly a T-junction gradient generator.

In this embodiment, the T junctions at the end of the microchannel are the convection units. In order to decouple convection through the side channel from diffusion across the main channel, bulk flow rates through the inlet and the outlet have to be matched. Pretending that one convection unit is the carrier of a constant solute concentration while the other unit carries buffer, this allows to create a gradient across the microchannel which remains constant as long as the flows and concentrations of solute at each convection unit remain the same.

The inventors validated this device in cell lines and on a patient tumor samples from human Gastrointestinal Stromal Tumor (GIST). GIST are genomically-driven neoplasms clinically treated with small molecule inhibitors targeting KIT and PDGFRA receptor tyrosine kinases. This novel technological approach permits a non-invasive, fast and accessible evaluation of therapies directly on patient-isolated cells (even from non-surgically obtained biopsies) in a precise and highly reproducible manner. Furthermore, due to the full integration of this assay inside a microfluidic chip, these analyses can be standardized to require minimal handling, enabling its future use as a routine assay at hospitals.

Moreover, the versatility of this microchip allows patient's tumor monitorization to adapt the therapeutic strategy throughout disease progression.

With this novel methodology will foster precision medicine to overcome cancer resistance to therapy and avoid relapse, by enabling the clinical implementation of the functional assay for a continuous personalized cancer treatment.

Since the equipment needed to perform this assay only requires an unsophisticated microfluidic pump and a fluorescent read-out, this new technology can be easily integrated into a ready-to-use medical device for *in situ* analyses at the hospital.

### Methods

In a third aspect, the present invention provides a method for predicting the cytotoxicity of a therapeutic agent towards a cell.

In a fourth aspect, the present invention provides a method for predicting cell protection of a therapeutic agent towards a cell.

All the embodiments provided above under the first or second aspect of the invention are also embodiments of the method of the third and fourth aspects of the invention.

In a first step, the methods of the invention comprises the contacting of an isolated test cell population with the test therapeutic agent.

The test cell population is isolated from the subject using any routine technique and is cultured using well-recognized and standardized culture media.

Since the cell requirement is lower than current technologies, the method of the invention could also be done in non-surgically obtained biopsies such as fine-needle aspirates. From a clinical standpoint this approach may represent a breakthrough on how to treat cancer patients and can greatly improve clinical outcomes, even for the most complicated cases

In one embodiment of the method of the third and fourth aspects of the invention, optionally in combination with any of the embodiments provided above or below, the cell is from a subject known to have or is suspected of having cancer. The subject is preferably a mammal. The mammal is, e.g., a human, non-human primate, mouse, rat, dog, cat, horse, or cow. In an alternative embodiment, optionally in combination with any of the embodiments provided above or below, the subject has been previously diagnosed as having cancer, and possibly has already undergone treatment for cancer. Alternatively, the subject has not been previously diagnosed as having cancer.

Then, the test cell population is exposed to the therapeutic agent.

In one embodiment of the method of the third and fourth aspects of the invention, optionally in combination with any of the embodiments provided above or below, the therapeutic agent induces apoptosis. In one embodiment of the method of the third and fourth aspects of the invention, optionally in combination with any of the embodiments provided above or below, the therapeutic agent is a chemotherapeutic agent. In another embodiment of the method of the third and fourth aspects of the invention, optionally in combination with any of the embodiments provided above or below, the therapeutic agent is a targeted chemotherapeutic agent. In another embodiment of the method of the third and fourth aspects of the invention, optionally in combination with any of the embodiments provided above or below, the targeted chemotherapeutic agent is a kinase inhibitor, such as an inhibitor of MEK1 and MEK2 kinase activity, an EGFR tyrosine kinase inhibitor or an inhibitor of kinases such as BCR-ABL, ABL, and KIT. In another embodiment of the method other therapeutic agents are chemotherapeutic agents such as doxorubicin, cisplatin, 5-FU and other cytotoxic agents used as anti-cancer agents. In another embodiment of the method other therapeutic agents are antibodies, including immune checkpoint inhibitors, such as pembrolizumab, rituximab, trastuzumab and others. In another embodiment of the method other therapeutic agents are engineered immune cells such as chimeric antigen receptor (CAR) T cell therapies.

Next, the test cell population, already exposed to the test therapeutic agent, is placed in contact with one or more BCL-2 family apoptotic inhibitors referred and defined in previous embodiments. This step is performed using routine means in the state of the art, using experimental conditions which are well-established or which can be easily optimized by those skilled in the art. An illustrative way is provided below, in the section of examples, wherein cells are in contact with the combination at 37°C for a suitable period of time which guarantees the induction of the apoptosis.

As it has been explained above, in the context of the invention, the expression "anti-apoptotic BCL-2 family inhibitors" encompasses any small molecule (with a molecular weight below 1.9, below 1.8, below 1.7, below 1.6, below 1.5, below 1.4, below 1.3, below 1.2, below 1.1 or below 1.0 KDa) which directly or indirectly inhibits BCL-2 function or induces its elimination. Among the potential targets there can be found the pro-apoptotic members BAX, BAK1, BIM, BID and BBC3 (commonly referred to as PUMA), and anti-apoptotic members of the same family, such as BCL-2, BCL-XL, BCL-W, BCL-2- A1 and MCL1. BH3-only proteins are a subclass of pro-apoptotic BCL-2 proteins that contain only one BCL-2 homology domain and can be divided into activators and sensitizers. Activator proteins (BIM, BID and PUMA) bind and directly activate BAX and/or BAK1, the central effectors of apoptosis, which subsequently undergo conformational changes and oligomerize, forming a pore in the mitochondrial membrane and causing MOMP. The function of sensitizer proteins (BAD, PMA- induced protein 1 (commonly referred to as NOXA), BIK, BMF and HRK) is that of inhibiting anti- apoptotic proteins and/or displacing activators (such as BIM or monomers of BAX or BAK1) that could ultimately lead to apoptosis. Anti- apoptotic members of the BCL-2 family have four BCL-2 homology domains forming a binding groove that sequesters activators or sensitizers as well as BAX and BAK1. The BH3- only proteins PUMA, NOXA, BID and BIM have a strong affinity for and inhibit MCL1, whereas BAD has a stronger affinity for BCL-2, BCL-W and BCL-XL than for MCL1.

There are well-known molecules modulating the above targets. In addition to the already mentioned above, further includes HA14-2, Obatoclax, Gossypol (AT-101), Oblimersen, ABT-737, ABT-263 (navitoclax), ABT-199 (venetoclax), APG-1252, APG-2575, AZD0466, BGB-11417, NU-0129, S55746/BCI-201, SPC2996, WEHI-539, A1155463, A1331852, DT2216, S-055746, A-1210477, AMG-176, AMG-397, AZD-5991, PRT1419, S64315/MIK665, S63845, MIM1, VU661013, GDC-0941, and others.

In one embodiment of the methods of the third and fourth aspects of the invention, optionally in combination with any of the embodiments provided above or below, the priming value is determined at different concentrations of the one or more anti-apoptotic BCL-2 family inhibitors, thus obtaining a priming curve (dose-response) for the test sample and for the reference sample. In this embodiment:
- when the priming curve of the test sample is shifted to the left with respect to the priming curve of the reference sample, this will indicate that the tested therapeutic agent is cytotoxic for the cell; and
- when the priming curve of the test sample is shifted to the right with respect to the priming curve of the reference sample, this will indicate that the tested therapeutic agent protects the cell.

In one embodiment of the step (b), optionally in combination with any of the embodiments provided above or below, the cell population is contacted to the combination as defined in the first aspect of the invention or in embodiments thereof.

Step (c) of the method of the third or fourth aspect of the invention determines apoptotic cell death in the sample. There are many protocols well-established and known for those skilled in the art allowing to determine the degree of apoptosis in a cell (cf. Banfalvi G. "Methods to detect apoptotic cell death", Apoptosis, 2017 Feb;22(2):306-323), most of them based on detecting abnormalities in cell membranes (such as plasmatic or mitochondrial membranes).

In one embodiment of the third aspect of the invention, optionally in combination with any of the embodiments provided above or below, step (c) comprises or consists of measuring the mitochondrial outer membrane permeabilization in the test cell population.

Outer membrane permeabilization is measured by a number of methods, for example, by loss of mitochondrial membrane potential. Loss of mitochondrial membrane potential is measured for example by measuring the release of molecules from the mitochondrial inter-membrane space. Examples of molecules that can be measured include cytochrome c, SMAC/Diablo, Omi, adenylate kinase-2 or apoptotic- inducing factor (AIF).

Release of molecules from the mitochondrial inter-membrane space can be measured by methods know in the art. For example, by using antibodies to the molecules to be measured, i.e., antibodies to cytochrome c, SMAC/Diablo, Omi, adenylate kinase-2 or apoptotic- inducing factor (AIF). Detection can be for example, by ELISA, FACS, immunoblot, immunofluorescence, or immunohistochemistry.

In one embodiment of the third aspect of the invention, optionally in combination with any of the embodiments provided above or below, step (c) comprises or consists of determining the % of cytochrome C released from the mitochondria.

In addition to measuring molecules that get released from the mitochondrial space, other intracellular and extracellular markers can be measured. This allows for the ability to discriminate between subpopulations of cells.

Alternatively, outer membrane permeabilization can be determined treating the cells, previously to step (c), with a potentiometric or radiometric dye.

Advantageously, a titration curve could be generated comparing treated vs reference cells to evaluate therapies' capacity to induce apoptosis.

In view of the above, in one embodiment of the third aspect of the invention, optionally in combination with any of the embodiments provided above or below, previous to step (c) the cell population is firstly in contact with a potentiometric or radiometric dye. Illustrative non-limitative examples of potentiometric dyes are hydrorhodamine 123, or tetramethylrhodamine methyl ester (TMRM) or tetramethylrhodamine ethyl ester (TMRE); particularly it is tetramethylilrhodamine (TMRE).

JC-1 is a lipophilic, cationic dye that enters mitochondria in proportion to the potential across the inner mitochondrial membrane. JC-1 exists as a monomer at low membrane concentrations). However, JC-1 accumulated in the mitochondrial matrix under conditions of higher mitochondrial potentials. At these higher concentrations, JC-1 forms red- fluorescent "J- aggregates". As a monomer the dye has an absorption/emission maxima of 527 nm while at high membrane potential the emission maximum is 590 nm. Thus, ratio measurements of the emission of this cyanine dye can be used as a sensitive measure of mitochondrial membrane potential. The dye allows for a dual measurement of dye concentration that does not require the measurement of a nuclear or cytoplasmic reference value. Studies using isolated mitochondria have shown that the 527 nm emission from monomeric JC-1 increases almost linearly with membrane (M) potentials ranging from 46 to 182 mV, whereas the 590 nm J-aggregate emission is less sensitive to M values less negative than 140 mv and is strongly sensitive to potential values in the range of 140 to 182 mV (Di Lisa et al., 1995). Optical filters designed for fluorescein and Tetramethylrhodamine ethyl ester (TMRE) can be used to separately visualize the monomer and J-aggregate forms, respectively. Alternatively, both forms can be observed simultaneously using a standard fluorescein longpass optical filter set.

Dihydrorhodamine 123 an uncharged, nonfluorescent agent that can be converted by oxidation to the fluorescent laser dye rhodamine 123 (R123).

The measuring of the amount of mitochondrial outer membrane permeabilization from the test population and the reference one can be made in parallel. No consecutive order of the steps is mandatory.

Alternatively, step (c) comprises or consists of measuring the activation of caspases.

Caspases (cysteine-aspartic proteases) are involved in the early stages of apoptosis. Activation of caspases represents the key event in the early apoptotic process. The molecular events leading to caspase activation have been utilized in cell-free systems for the detection of apoptosis generated in the mitochondrial death pathway. Caspase activation can be detected by in vitro enzyme assays. Western blot mixtures have been designed to detect proteins synthesized in response to apoptotic stimuli, such as caspases

Quantitative reverse transcription PCR (RT-qPCR) is used when the starting material is RNA. For RT-qPCR analysis of gene expression activated (cleaved) effector caspases 3 and 7, activated initiator caspases 2, 8 and 9, APAF, Bax, Bak, Bid, PARP are recommended. Beside the early detection of apoptosis by western blot analysis, caspase activity can be detected by the cleavage of an in vivo caspase substrate, flow cytometry, fluorescence and light microscopy, and by fluorescence ELISA methods. Fluorometric substrates or antibodies preferentially to caspase 3 and caspase 7 generate fluorescence proportional to caspase activation. Caspase-cleaved cytokeratin 18 (M30) is a specific antibody at the early stage of apoptosis that does not react with intact or necrotic cells. The detection of M30 is a reliable indicator of apoptosis in epithelial cells. Another antibody, anti-PARP may be used to detect PARP fragments and intact PARP from apoptotic and healthy cell extracts on a western blot. Caspase cleavage of PARP can be measured by western blot, immunohistology and immunoprecipitation. p53, annexin V and M30 proteins are detected in a similar manner.

Alternatively, step (c) can be performed by measuring the extracellular phosphatidylserine.

Annexin A5 or annexin V is a member of the calcium-dependent phospholipid-binding proteins that adheres specifically to phosphatidyl serine (PS). In healthy cells PS is located in only the cytosolic side of the plasma membrane. In the early stage of apoptosis, the asymmetric distribution of PS is lost and PS translocates to the extracellular leaflet of the membrane. The presence of PS in the extracellular side of the cell membrane can be detected with fluorescently labelled annexin V. Suitable dyes are impermeable dyes, such as propidium iodide (PI), trypan blue, 7-amino-actinomycin (7-AAD).

The amount of mitochondrial cell apoptotic death obtained from the test cell population is compared to the amount obtained from a reference cell population that has not been contacted with the therapeutic agent.

Any statistical method can also be used in the management of the measures obtained.

The reference cell population can be a test cell population isolated from the same subject but which has not been in contact with the therapeutic agent. Alternatively, the reference cell population a cell population of the same nature as the test one but from a healthy subject. By the expression "the same nature" the invention refers to the sample is isolated from the same organ/nature of sample.

In one embodiment of the method of the third aspect of the invention, a kit as defined in the second aspect of the invention, alone or in combination with any of the embodiments provided above, is used.

In another embodiment, the method of the third aspect of the invention comprises the use of a microfluidic device as defined above. In this embodiment, step (a) can be performed either inside or outside the microfluidic device. In one embodiment, step (a) is performed outside the microfluidic device.

Following with the method, step (b) is performed in the microfluidic device and comprises the filling of wells with the resulting cells and the perfusion of the one or more BCL-2 anti-apoptotic family inhibitors in a buffer. In one embodiment, optionally in combination with any of the embodiments provided above or below, the perfusion of the BCL-2 inhibitors is performed by creating a gradient of concentration. In another embodiment, optionally in combination with any of the embodiments provided above or below, the gradient of concentration is made by perfusing the culture medium alone by one of the inlets of the device and the culture medium+the one or more BCL-2 inhibitors through a second inlet of the device.

Steps (c) to (e) are performed as explained in detail above.

In a further embodiment of the method of the third aspect of the invention, optionally in combination with any of the embodiments provided above or below, the cell population is a cancer cell population and the therapeutic agent is a chemotherapeutic agent as explained in detail above.

### Examples

### Cell culture and treatments

GIST-T1 and GIST-T1/670 were kindly provided by Dr. Cesar Serrano from Vall d'Hebron Institute of Oncology. These cells were cultured in IMDM medium (ThermoFisher Scientific, Waltham, MA, USA) supplemented with 15% of fetal bovine serum (FBS) (10270106, ThermoFisher Scientific), 1% of penicillin/streptomycin (15140122, Gibco ThermoFisher Scientific) and 1% of L-glutamine (25030024, ThermoFisher Scientific) at 37°C and 5% of CO2.

NALM-6 and SEM cell lines were kindly provided by Prof. Pablo Menéndez laboratory at the Josep Carreras Leukaemia Research Institute. Both cell lines were cultured in RPMI 1640 medium (31870, Thermo Fisher, Gibco, Paisley, Scotland) with 10% of heat-inactivated fetal bovine serum (10270, Thermo Fisher, Gibco), 1% of L-glutamine (25030, Thermo Fisher, Gibco) and 1% of penicillin/streptomycin (15140, Thermo Fisher, Gibco). Cells were maintained inside a humidified incubator at 37°C and 5% of CO2.

Imatinib, gefitinib were obtained from LC Laboratories (Woubourn, MA, USA); trametinib from SelleckChem (Munich, Germany) and ABT-199, A-1331852 and S6385 at MedChemExpress (Monmouth Junction, NJ, United States).

1mM of the combination of BH3 mimetics was prepared adding 0.1mM of ABT-199, 0.1mM of A-1331852 and 1mM of S63845.

### Dynamic BH3 profiling with flow cytometry (comparative purpose)

BCP-ALL and GIST cells were pre-treated for 16 hours with different anticancer drugs, stained with Zombie Violet to mark viable cells, cleaned with phosphate bovine saline (PBS) and separated in 9 different tubes resuspended in mannitol experimental buffer (MEB) (150 nM mannitol, 10 mM HEPES-KOH pH 7.5, 150 mM KCI, 1 mM EGTA, 1 mM EDTA, 0.1% BSA and 5 mM succinate) with 0.001% digitonin and 0.01, 0.03, 0.1, 0.3, 1, 3 and 10 µM of BIM peptide, 25 µM of alamethicin and a DMSO only control. After 1 hour of incubation at RT, cells were fixed with formaldehyde 8%, neutralize with N2 buffer (1.7 M tris base, 1.25 M glycine at pH 9.1) and stained with intracellular staining buffer (1% Tween20, 5% BSA in PBS) with 1:1000 of cytochrome C antibody conjugated with Alexa Fluor 647^{®}. After overnight incubation at 4 °C, results were obtained in a LSRII flow cytometer and processed using FlowJo software, following manufacturer's instructions. Gates for % cytochrome c retained were made in the untreated condition and applied to all the conditions.

### Real-time tracking of apoptosis (RTA) with flow cytometry (invention)

Cells were seeded in a 12-well plate and treated for 16 hours with different anticancer drugs. After incubation, cells were trypsinized, stained with Zombie Violet and cleaned with PBS. After that, cells were resuspended in the appropriate media without FBS and 25uL of every cell solution was added into a 96-well plate with 25uL of a solution of media without FBS and different dilutions of the combination of BH3 mimetics (3uM, 1uM, 0.3uM, 0.1uM, 0.03uM, 0.01uM, 0.003uM and only DMSO). Plates were incubated at 37°C for 1 hour in case of the BCP-ALL cell lines and 3 hours in case of the GIST cell lines and stained following the protocol of: fixation with 8% PFA, neutralization with N2 buffer and stained using antibodies against cytochrome c. Since BCP-ALL are non-adherent cells, after overnight incubation at 4°C, the results were obtained using a LSRII flow cytometer and processed using FlowJo software. Gates for % cytochrome c retained were made in the untreated condition and applied to all the conditions.

### RTA with fluorescence microscopy

100,000 cells were resuspended in 1mL of complete media, treated with the corresponding anticancer drug and 100uL of the cell solution were seeded in 8 wells of a 96-well plate for every treatment condition. Cells were incubated for 16 hours. After the incubation cells were stained with 2uM of Calcein AM and 100nM of TMRE in complete media (media used in cell culture) for 30 minutes at 37°C, cleaned with PBS and exposed to the same conditions of the combination of BH3 mimetics as the flow cytometry version. The plates were incubated for 3 hours at 37°C and images were taken with a Nikon Eclipse Ti microscope. Using CellProfiler cells were automatically identified using the Calcein AM field and the intensity of TMRE was quantified to differentiate apoptotic from non-apoptotic cells applying a threshold of intensity at the 90% percentile of fluorescent intensity in the control condition.

### SU8 mold fabrication

To develop the mold, a silicon wafer (4" n-type <100>, MicroChemicals GmbH, Ulm, Germany) was cleaned in a PCD-002-CE Plasma Cleaner (Harrick Plasma, Ithaca, NY, USA) for 20 minutes at 6.8W and heated in a hotplate at 95°C for 5 minutes. Next, SU-8 photoresist (2100, MicroChem Lab, Westborough, MA, USA) was spin coated over the wafer (first at 500 rpm for 5 seconds with an acceleration of 100 rpm/s followed by 3000 rpm for 30 seconds with an acceleration of 401 rpm/s) obtaining a 100 µm thick layer. The wafer was soft-baked at 65°C for 5 minutes and 95 °C for 20 minutes for solvent evaporation. Patterning with the microfluidic chip design was obtained by energy radiation of 240 mJ/cm2 using a negative photoresist mask printed in high-quality acetate film. Photoresist was cross-linked by exposing the wafer to 65 °C for 5 minutes and 95 °C for 10 minutes in a hot plate. Then, labile photoresist was removed by immersion in SU-8 developer (Y020100, MicroChem Lab) for 10 minutes and washed with 2-propanol. Finally, the wafer was placed in a hot plate at 150 °C for 60 minutes with a final decrease until reaching room temperature, when they were silanized to obtain and hydrophobic surface.

### Fabrication of a microfluidic chip

To obtain a microfluidic chip, PDMS was prepared by mixing Sylgard 184 (Dow Corning, Midland, MI, USA) prepolymer with curing agent in a ratio 1:10 followed by a degasification in a void chamber for 1 hour. Three different layers were prepared calculating the polymer volume to obtain the desired thickness of PDMS layer. For the first 2 mm thick layer, PDMS prepolymer was poured onto the SU8 master mold with design motifs fixed inside a plastic Petri dish; for the second 1 mm thick layer, prepolymer was dispensed directly into an empty Petri dish; and for the third layer, a clean 75 x 50 mm glass slide (CLS294775X50, Sigma-Aldrich) was pressed against uncured polymer mix to obtain a thin layer of PDMS over the glass. PDMS was placed at room temperature on a flat surface followed by 4 hours at 85 °C. PDMS layers were then carefully peeled off and holes for inlets and outlets were punched in the 2 mm layer using a 0.5 mm biopsy punch. Both layers (2 and 1 mm thick) were irreversibly bound by plasma activation, baked for 4 hours at 85 °C and punched using a 4 mm biopsy punch to create the cell chamber wells, the resulting 3 mm thick PDMS layer was irreversible bound to the thin PDMS layer over the glass slide and heated at 85 °C for 4 hours. Finally, a second glass slide was used to cover the wells during the experiments.

### RTA with microfluidics

Chips were placed in an oven at 85 °C for 1 hour. 10 µL of sterile MiliQ water with 15 µg/mL of poly-L-lysine was added to each well and incubated at 37 °C for 40 minutes to coat the well surface, followed by a cleaning step with MiliQ water. 100.000 cells were resuspended in 600 µL of complete media (media used in cell culture) and separated in 3 different tubes, where the appropriate concentration of treatment was added. 35 µL of the cell suspension was added to every well and the chip was incubated at 37 °C for 16 hours. After the incubation, the wells were refilled with complete media and sealed with a glass slide secured with plastic alligator clips. Then, complete media with 100 nM of TMRE and 2 µM of Calcein AM was perfused through the two inlets at a pressure of 300 mbar for 15 minutes using the P2SC pump and chips were incubated at 37 °C for 40 minutes. Next, complete media was perfused through one inlet at 200 mbar for 20 minutes, while in the other inlet the same buffer with a specific concentration of the combinations of BH3 mimetics. The chip was incubated for 2 hours at room temperature before acquiring images using the Nikon fluorescence microscope.

### Characterization of the gradient

A solution containing 10 mg/mL of bovine serum albumin (BSA) (A3059, Sigma-Aldrich) and 428 µL/mL of blue food dye (Vahine, Catalonia, Spain) in MiliQ water was perfused through the first inlet of the chip and only MiliQ water through the second inlet at 200 mbar using a Precision Pressure Control System P2CS pump (Biopyhysical tools, Leipzig, Germany). Liquid coming out from the outlets was collected in Eppendorfs. Concentration of BSA was measured using a PierceTM BCA protein quantification kit (23225, ThermoFisher Scientific) and absorbance in the blue 640 nm wavelength was measured using a Benchmark Plus Microplate Reader (4100172C, Bio-Rad, California, USA). Fluorescence gradient characterization was performed injecting 25 µg/mL of fluorescein (F2456, Sigma-Aldrich) in 10 mM NaOH and taking images with a 436 ZEISS Axio Observer Z1/7 microscope.

### Statistical analysis

All results were expressed as the mean ± S.E.M. of at least three biologically independent replicates. Every condition was compared to the control condition using an unpaired t-test and marked as statistically significant (*) when p-value was lower than 0.05. GraphPad Prism 9 was used to perform statistical analyses and represent the results.

### Results

### Real-time tracking of apoptosis identifies effective treatments in liquid tumors

The inventors compared the efficiency in priming the apoptosis using the combination of the invention with the methodology already reported in the prior art WO2014047342 (hereinafter also referred as "standard DBP").

In the NALM-6 cell line, a similar dose-response curve to the one in standard DBP was obtained (Figure 1A), which shows that the titration of the combination of BH3 mimetics has a similar effect as the BIM peptide after treating the cells with digoxin. Importantly, in cells treated with trametinib lower concentrations of the combination of BH3 mimetics was needed to start the apoptotic process, while in the case of imatinib, the same curve as the control condition was obtained (Figure 1C).

In SEM cells similar results were also obtained, where SEM cells treated with sunitinib were more sensitive to the combination of BH3 mimetics of the invention, showing an increase in apoptotic priming that correlates with future cytotoxicity (Figure 1B and 1D).

Results of these type of assays can also be expressed as Δ%priming, which represent the difference in apoptotic priming between the treated condition and the control condition when the dose-response curve starts to decay. Comparing the results of DBP and RTA, an increase in Δ%priming was obtained after treatment with trametinib in NALM-6 and sunitinib in SEM, while in both cells lines after imatinib there was not an increase in apoptotic priming (Figure 1E an 1F). With these results it was demonstrated that the new RTA protocol could identify effective treatments in BCP-ALL cell lines with the same or higher effectivity as standard DBP with the remarkable advantage that cells are not required to be subjected to any lytic step.

### In solid tumors, RTA can be used with flow cytometry and fluorescent microscopy

To demonstrate that the new assay also worked in adherent cells, two Gastrointestinal Stromal Tumor (GIST) cell lines, GIST-T1 and GIST-T1/670 were used. As previously published, GIST-T1 cell lines are sensitive to imatinib treatment, whereas GIST-T1/670 acquired resistance to this inhibitor.

Again, it was performed standard DBP with GIST-T1 after treatment with imatinib and gefitinib as a negative control.

Pre-treatment with imatinib caused a decrease in the concentration of BIM peptide needed to start the process of apoptosis compared to the untreated condition, while treatment with gefitinib showed a similar profile as the control (Figure 2A).

In the case of the GIST-T1/670 cell line the three experimental conditions had the same dose-response curve since neither imatinib nor gefitinib is effective in these cells (Figure 2B). A dose-response curve was obtained when RTA was performed with the combination of BH3 mimetics of the invention and the flow cytometry readout. Similarly to the results in BCP-ALL, treatment with imatinib in GIST-T1 caused a decrease in the concentration of the inductor of apoptosis to start the process, showing an increase in apoptotic priming after treatment with an effective drug (Figure 2C).

Again, treatment with imatinib in the resistant cell line GIST-T1/670 or gefitinib in both cell lines produced the same curve as the control conditions (Figure 2D).

The new protocol provided by the present invention avoids the permeabilization of the cells with digitonin, but at the end of the incubations cells are fixed to allow immunostaining of cytoc.

To improve it, it has been developed a new methodology based on TMRE, a fluorescent dye that accumulates in healthy mitochondria (non-apoptotic cells) but loses the fluorescence when mitochondria are permeabilized (apoptotic cells). To prove that this microscopic version achieves similar results, we repeated the experiments incorporating this die. It was found that GIST-T1 cells provided a dose-response curve based on the intensity of TMRE that differentiated apoptotic from non-apoptotic cells. Importantly, pre-treatment with imatinib caused a shift of the curve identifying an increase in apoptotic priming, while in gefitinib-treated cells (negative control) this was not observed (Figure 2E). As expected, in the case of GIST-T1/670 cells, both treatments produced an unaltered curve, similar to the control condition (Figure 2F). When the Δ%priming was quantified for all three methods, similar results were obtained in all cases, with a clear increase in apoptotic priming after imatinib treatment in GIST-T1 cells and no significant changes in the other conditions (Figure 2G, and 2H). The RTA using flow cytometry also worked° in adherent cell lines of GIST.

### RTA can be performed in a microfluidic chip

One of the main limitations of both the classic DBP and initial RTA experiments is that both required a high number of viable cells to perform the assay.

The inventors incorporated the combination of the invention into a microfluidic chip that greatly reduces the number of cells required and allows to automatize the process helping its implementation in the clinic.

The microfluidic device was able to identify effective treatments using the combination of BH3 mimetics as an apoptotic inductor but using fewer cells. The combination of RTA and the microfluidic device could be further developed to generate a kit to identify anticancer drugs in patient samples *in situ* at the hospital.

## Claims

1. A combination comprising a BCL-2 inhibitor, a BCL-xL inhibitor and a MCL-1 inhibitor.

2. The combination of claim 1, which consists of a BCL-2 inhibitor, a BCL-xL inhibitor and a MCL-1 inhibitor.

3. The combination of any one of the preceding claims, which comprises or consists of ABT-199, A-1331852 and S63845.

4. A kit comprising the combination of inhibitors as defined in any one of the claims 1-3.

5. The kit of claim 4, which is a microfluidic device.

6. The kit of claim 5, which comprises means to generate within the device a gradient, particularly a logarithmic gradient.

7. The kit of claim any one of the preceding claims 6-7, which comprises a T-junction gradient generator.

8. The combination of any one of the claims 1-3 or the kit of any one of the claims 4-7, which comprises one or more further anti-apoptotic BCL-2 family inhibitors having a molecular weight below 2 KDa, particularly below 1.5 KDa, particularly below 1 KDa.

9. A method for predicting the cytotoxicity of a therapeutic agent towards a cell, the method comprising:
a) contacting an isolated test cell population to the test therapeutic agent;
b) contacting the cell population resulting from step (a) to one or more anti-apoptotic BCL-2 family inhibitors, the one or more anti-apoptotic BCL-2 family inhibitors having a molecular weight below 2 KDa, particularly below 1.5 KDa, particularly below 1 KDa ;
c) determining an apoptotic priming value;
d) obtaining a reference apoptotic priming value by contacting another isolated cell population sample from the subject with the one or more anti-apoptotic BCL-2 family inhibitors used in previous step (b); and
e) comparing both priming values;
wherein:
- step (d) is performed in the absence of the therapeutic agent;
- the process is performed in the absence of a cell permeabilizing agent; and
wherein a reduction in the priming value from the test cell portion compared to that in the reference cell portion indicates that the therapeutic agent is cytotoxic for the cell.

10. A method for predicting cell protection of a therapeutic agent, the method comprising:
a) contacting an isolated test cell population to the test therapeutic agent;
b) contacting the cell population resulting from step (a) to one or more anti-apoptotic BCL-2 family inhibitors, the one or more anti-apoptotic BCL-2 family inhibitors having a molecular weight below 2 KDa, particularly below 1.5 KDa, particularly below 1 KDa;
c) determining an apoptotic priming value;
d) obtaining a reference apoptotic priming value by contacting another isolated cell population sample from the subject with the one or more anti-apoptotic BCL-2 family inhibitors used in previous step (b); and
e) comparing both priming values;
wherein:
- step (d) is performed in the absence of the therapeutic agent;
- the process is performed in the absence of a cell permeabilizing agent; and
wherein an increase in the priming value from the test cell portion compared to that in the reference cell portion indicates that the therapeutic agent protects the cell.

11. The method of any one of the claims 9-10, wherein the priming value is determined with different concentrations of the one or more anti-apoptotic BCL-2 family inhibitors, thus obtaining a priming curve for each of the test sample and the reference sample.

12. The method of claim 11, wherein:
when the priming curve of the test sample is shifted to the left with respect to the priming curve of the reference sample, this will indicate that the tested therapeutic agent is cytotoxic for the cell; and
when the priming curve of the test sample is shifted to the right with respect to the priming curve of the reference sample, this will indicate that the tested therapeutic agent protects the cell.

13. The method of any one of the claims 9-12, wherein steps (c) and (d) are performed by measuring the mitochondrial outer membrane permeabilization in the test and reference cell populations.

14. The method of any one of the claims 9-13, wherein the measuring of the mitochondrial outer membrane permeabilization comprises measuring the release of molecules from the mitochondrial intermembrane space; particularly comprises the release of one or more of the following molecules from the mitochondrial intermembrane space: cytochrome c, SMAC/Diablo, Omi, adenylate kinase-2 or apoptotic- inducing factor (AIF); particularly the release of cytochrome c.

15. The method of any one of the claims 9-14, wherein the cell population is a cancer cell population and the therapeutic agent is a chemotherapeutic agent; particularly a targeted chemotherapeutic agent; particularly a kinase inhibitor.
